# EUROPÄISCHE PATENTSCHRIFT

(11) **EP 1 483 252 B1**
(45) Veröffentlichungstag und Bekanntmachung des Hinweises auf die Patenterteilung: **20.07.2005**
(21) Anmeldenummer: 03709732.6
(22) Anmeldetag: 28.02.2003
(51) Int. Cl.: C07D 307/08, B01D 3/14

(54) **VERFAHREN ZUR DESTILLATIVEN AUFARBEITUNG VON TETRAHYDROFURAN**
METHOD FOR PURIFYING TETRAHYDROFURAN BY DISTILLATION
PROCEDE DE PURIFICATION PAR DISTILLATION DE TETRAHYDROFURANNE

(30) Priorität: 02.03.2002 DE 10209632
(43) Veröffentlichungstag der Anmeldung: 08.12.2004
(73) Patentinhaber: BASF AKTIENGESELLSCHAFT, 67056 Ludwigshafen (DE)
(72) Erfinder: WINDECKER, Gunther, 67067 Ludwigshafen (DE); WECK, Alexander, 67251 Freinsheim (DE); FISCHER, Rolf-Hartmuth, 69121 Heidelberg (DE); RÖSCH, Markus, 55276 Dienheim (DE); BOTTKE, Nils, 68165 Mannheim (DE); HESSE, Michael, 67549 Worms (DE); SCHLITTER, Stephan, 67117 Limburgerhof (DE); BORCHERT, Holger, 67591 Offstein (DE)
(86) Internationale Anmeldenummer: PCT/EP2003/002045
(87) Internationale Veröffentlichungsnummer: WO 2003/074507

(56) Entgegenhaltungen:
- DE-A- 3 726 805
- DE-A- 10 021 703
- US-A- 4 332 645

## Beschreibung

Die Erfindung betrifft ein kontinuierliches Verfahren zur destillativen Reinigung von Tetrahydrofuran (THF).

Die Herstellung von THF durch Dehydratisierung von 1,4-Butandiol an sauren Katalysatoren wird beispielsweise in DE-A 29 30 144 beschrieben und führt zu THF-Rohproduktion mit Wassergehalten von 18 bis 28 Gew.-% und Gehalten bis zu 5 Gew.-% von durch die Synthese bedingten Verunreinigungen wie 2,3-Dihydrofuran, 2- und 3-Methyltetrahydrofuran.

Für die Gewinnung von reinem THF aus derartigen Rohprodukten ist aus DE-A 37 26 805 ein Verfahren zu destillativen Reinigung von rohem wasserhaltigen THF bekannt, bei dem das THF durch drei Destillationskolonnen geleitet wird, wobei ein Seitenabzug der ersten Kolonne in die zweite geleitet wird, das Kopfprodukt der dritten Kolonne in die erste Kolonne zurückgeführt wird, am Kopf der ersten Kolonne Destillat entnommen wird und man das reine THF aus dem Seitenabzug der dritten Kolonne gewinnt.

Die Herstellung von THF durch Gasphasenhydrierung von Maleinsäureanhydrid (MSA) ist eine seit vielen Jahren bekannte Reaktion und ist beispielsweise in JP-B 2639463 und JP-B 2639464 beschrieben. Die erhaltenen wasserhaltigen Rohprodukte unterscheiden sich von den durch Butandiol-Dehydratisierung erhaltenen insbesondere durch ihren höheren Anteil an Komponenten, deren Siedepunkt sich vom Siedepunkt des THF nur wenig unterscheiden, sogenannten engsiedenden Komponenten. Unter den engsiedenden Komponenten des THF's sind insbesondere Methanol, Ethanol und Butyraldehyd, beziehungsweise deren Azeotrope mit THF und/oder Wasser zu nennen.

Die wasserhaltigen THF-Rohprodukte der Gasphasenhydrierung von MSA werden durch das aus DE-A 37 26 805 bekannte Verfahren nicht ausreichend aufgereinigt, um die insbesondere bei der Weiterbearbeitung des THF's, beispielsweise zu PTHF, an THF gestellten Reinheitsanforderungen zu erfüllen.

Es stellte sich daher die Aufgabe, ein verbessertes Verfahren zu finden, welches wirtschaftlich die Gewinnung von THF in hoher Reinheit aus wasserhaltigen THF-Rohprodukten ermöglicht. Dabei soll es unabhängig von der Herstellungsweise möglich sein, diese Reinheiten zu erreichen.

Diese Aufgabe wird gelöst durch ein Verfahren zur destillativen Reinigung von rohem wasserhaltigen Tetrahydrofuran, bei dem an das rohe Tetrahydrofuran durch drei Destillationskolonnen leitet, Wasser aus dem Sumpf der ersten Kolonne abzieht, wasserhaltiges Tetrahydrofuran vom Kopf der zweiten Kolonne in die erste zurückführt, einen Seitenabzug der ersten Kolonne in die zweite Kolonne leitet, das Sumpfprodukt der dritten Kolonne in die erste Kolonne zurückführt, am Kopf der ersten Kolonne ein Destillat entnimmt, das dadurch gekennzeichnet ist, dass man einen Seitenabzug der zweiten Kolonne in die dritte Kolonne leitet und das reine Tetrahydrofuran als Kopfprodukt der dritten Kolonne gewinnt.

Das erfindungsgemäße Verfahren ist auf rohes, wasserhaltiges THF, das durch unterschiedlichste Herstellungsverfahren erzeugt wurde, anwendbar. Besonders geeignet ist es für THF-Rohprodukte, die durch MSA-Hydrierung entstanden sind. Es ermöglicht die Gewinnung eines hochreinen THF's, wobei die engsiedenden Komponenten sicher entfernt werden.

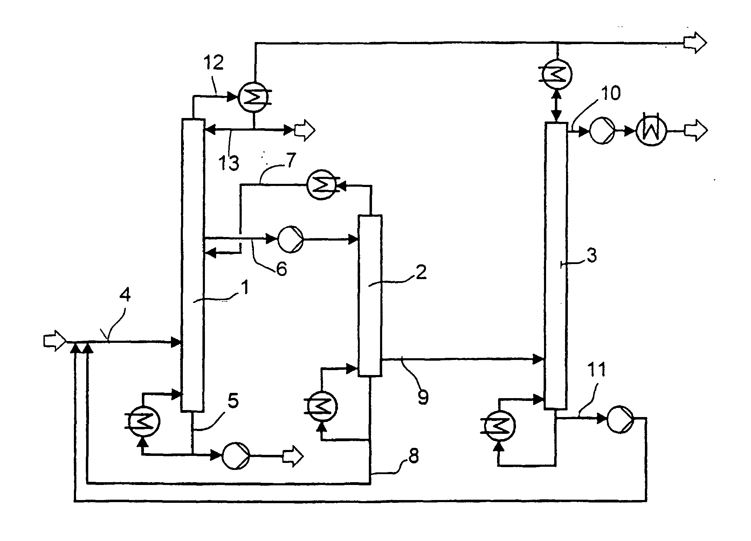

Nach dem neuen Verfahren nimmt man die destillative Reinigung des rohen wasserhaltigen THF's, das vorzugsweise durch Hydrierung von MSA erhalten wurde, in drei hintereinandergeschalteten Kolonnen so vor, wie es aus der Figur ersichtlich ist. Dabei werden die Kolonnen auf an sich übliche Weise betrieben, und zwar die erste Kolonne (1) mit Druck von 1,3 bar mit mindestens 10, bevorzugt 30 bis 70, besonders bevorzugt 45 bis 55. theoretischen Trennstufen und einem Rückflussverhältnis, bezogen auf den Seitenabzug (5) von 0,5 bis 5, die zweite Kolonne (2) mit mindestens 10, bevorzugt 30 bis 70, besonders bevorzugt 45 bis 55, theoretischen Trennstufen, einem Druck von 5 bis 10 bar, bevorzugt zwischen 7 und 9 bar, besonders bevorzugt 8 bar, und die dritte Kolonne (3) mit mindestens 10 theoretischen Trennstufen, bevorzugt 30 bis 70 Trennstufen, besonders bevorzugt 45 bis 55 theoretischen Trennstufen, bei einem Druck von 0,9 bis 2 bar, bevorzugt 1 bis 1,5 bar und bevorzugt 1,3 bar und einem Rückflussverhältnis von etwa 3,8.

Jeder der drei Kolonnen (1), (2) und (3) weist mindestens eine theoretische Trennstufe auf, welche dadurch gekennzeichnet ist, das der entstande Brüdenstrom und der flüssige Rücklauf der Stufe im Gegensromprinzip aneinander vorbeigeführt werden. Die Einbauten der Kolonnen (1), (2) und (3) können aus Füllkörpern, Gewebe- oder Blechpackungen oder Trennböden wie Ventil-, Tunnel- oder Siebböden bestehen. Eine Definition für eine theoretische Trennstufe kann beispielsweise E.-U. Schlunder, F. Thurner, Destillation, Absorption, Extraktion, Thieme Verlag 1986, Seite 66 und Seiten 131 - 132 entnommen werden.

Das rohe wasserhaltige THF, das durch Gasphasenhydrierung von MSA gewonnen wurde, besteht im allgemeinen aus 61 Gew.-% THF, 4 Gew.-% n-Butanol (n-BuOH), 0,7 Gew.-% Methanol (MeOH), 0,5 Gew.-% Ethanol (EtOH), 1 Gew.-% Propanol (ProOH), 400 ppm Gamma-Butyrolacton (GBL), 120 ppm Butyraldehyd (BA), 100 ppm Butylmethylether (BME), weiteren O-funktionalisierten CH-Verbindungen in Konzentrationen < 200 ppm, sowie Wasser.

Es wird seitlich über die Zuleitung (4) in die erste Kolonne geleitet. Die Zuleitung (4) befindet sich zweckmäßigerweise in der unteren Hälfte oberhalb des Sumpfes der Kolonne. Erfindungsgemäß wurde erkannt, dass der Zulauf zwischen 1 und 30 theoretischen Trennstufe, bevorzugt zwischen der 1 und 20 Trennstufe, besonders bevorzugt zwischen der 1 und 10 Trennstufe liegen sollte. Vom Sumpf der Kolonne werden Wasser und schwersiedende Komponenten, welche einen höheren Siedepunkt als THF aufweisen, wie gamma-Butyrolacten, Ethanol, Propanol, Butanol zusammen mit Wasser, ausgeschleust (5). Die Leichtsieder, welche einen niedrigeren Siedepunkt als THF aufweisen, wie Methanol, werden über Leitung (12) mit THF über Kopf gezogen, teilweise über einen Wärmetauscher kondensiert und als Rücklauf (13) wieder in Kolonne (1) gefahren. Die Kolonne (1) weist einen oberhalb der Zuleitung (4) angebrachten Seitenabzug (6) auf, durch den man ein bevorzugt flüssiges THF/Wasser-Gemisch, das jedoch gasförmig oder als Flüssigkeits/Gasgemisch vorliegen kann, entnimmt und unter Druckerhöhung über eine Pumpe seitlich in die Mitteldruckkolonne (2) einleitet, wobei der Zulauf dieses Stroms zwischen der Hälfte der theoretischen Kolonnenstufenzahl und dem Kopf der Kolonne liegt.

Der Seitenabzug (6) ist zwischen der 20. und 70. theoretischen Trennstufe der Kolonne (1), bevorzugt zwischen der 30 und 55 Trennstufe, besonders bevorzugt der 30. und 40. Trennstufe angeordnet.

In dem Seitenabzug (6) liegt THF zum Wasser in einem Gewichtsverhältnis von 13:1 bis 25:1, bevorzugt in einem Verhältnis von 15:1 bis 22:1 vor.

Der Zulauf des Seitenabzuggemisches (6) erfolgt im oberen Teil der Mitteldruckkolonne (2), zwischen der 30. und 70. theoretischen Trennstufe, bevorzugt zwischen der 40. und 60. Trennstufe, besonders bevorzugt zwischen der 50. und 60. Trennstufe. Unter Verschiebung des azeotropen Punktes des THF/Wassergemisch wird in der Mitteldruckkolonne (2) das Gemisch nochmals aufgetrennt. Vom Kopf dieser Mitteldruckkolonne wird wasserreiches THF über einen Wärmetauscher kondensiert und zwischen Seitenabzug (6) und dem Kolonnensumpf in die erste Kolonne zurückgeführt (7). Das Sumpfprodukt dieser Mitteldruckkolonne (2), das im wesentlichen wasserfrei ist, und aus THF und Schwersiedern wie beispielsweise Butyraldehyd, Butylmethylether und weitere hochsiedende O-funktionalisierte CH-Verbindungen besteht, wird über Leitung (8) zurückgeführt und kann mit rohen wasserhaltigen THF vermischt, erneut Kolonne (1) über Leitung (4) als Feed zugeführt werden.

Im Abtriebsteil der Mitteldruckkolonne (2) reichert sich THF auf über 99 Gew.-% an. Um die wasserdampfflüchtigen Komponenten nicht in die Kolonne (3) zu schleppen, wird ein THF-reicher Strom in bevorzugt flüssiger Form, der jedoch auch gasförmig oder als Flüssigkeits/Gasgemisch vorliegen kann, zwischen der Dampfphase des Sumpfes und der Hälfte der theoretischen Kolonnenstufenzahl an einem flüssigen Seitenabzug (9) kurz über dem Sumpf der Mitteldruckkolonne (2) entnommen.

Der der Kolonne (2) entnommene Seitenabzug (9) enthält 50 bis 100 Gew.-% THF, bevorzugt 80 bis 100 Gew.-%, besonders bevorzugt 95 bis 100 Gew.-% THF auf.

Der Seitenabzug (9) wird oberhalb des Sumpfes in Kolonne (3) eingespeist. Die Zulaufstelle liegt zwischen der 1. und 30. theoretischen Trennstufe, bevorzugt zwischen 1. und 15. Trennstufe, besonders bevorzugt zwischen 5. und 10. Trennstufe. Reines THF wird am Kopf der Kolonne (10) in flüssiger oder gasförmiger Form oder als Flüssigkeits/Gasgemisch, bevorzugt flüssig, abgezogen, während das flüssige Sumpfprodukt über eine Pumpe in Kolonne (1) rückgeführt wird.

Das erfindungsgemäße Verfahren wird nun in den nachfolgenden Beispielen näher erläutert.

### Beispiele

### Beispiel 1

Es wurde die durch die Figur dargestellte Destillationsapparatur verwendet. Sie besteht aus den drei Kolonnen (1), (2) und (3), von denen die Kolonne (1) 48 und die Kolonne (3) 45 theoretischen Böden aufweisen und bei 1,3 bar absolut betrieben werden. Kolonne (2), die bei erhöhtem Druck von 8 bar betrieben wird, hat 43 theoretische Böden.

In die Kolonne (1) wurde über die Zuleitung (4) ein wasserhaltiges THF-Gemisch mit 64,6 Gew.-% THF, 2,9 Gew.-% n-BuOH, 0,4 Gew.-% MeOH, 0,6 Gew.-% EtOH, 0,5 Gew.-% PrOH, 0,4 Gew.-% GBL, 124 ppm BA und 115 ppm BME. Der Rest sind weitere O-funktionalisierte CH-Verbindungen und größtenteils Wasser.

Gleichzeitig wurde der Kolonne (1) über Zuleitung (8) der Sumpfabzug der Kolonne (2) und über Leitung (11) der Sumpfabzug der Kolonne (3) zugeführt. Die Kolonne (1) wurde mit einem Seitenabzug (6), einem Kopfabzug (12) und einem Sumpfabzug (5) betrieben. Das Rücklaufverhältnis bezogen auf den Seitenabzug, betrug 1,1. Aus dem Sumpfabzug (5) wurde Wasser und Schwersieder abgeleitet.

Der Kolonne (1) wurde als Seitenabzug (6) ein wasserhaltiges THF mit einem Wassergehalt von 4 Gew.-% und einer THF-Konzentration von 82,5 Gew.-% entnommen. Dies entspricht einem THF:Wasser Verhältnis von 20,6.

Als Kopfabzug (12) wurden etwas über 0,05 % des Zulaufs entnommen. Der Kopfabzug hatte eine THF-Konzentration von 64 % und einen Methanolgehalt von 32 Gew.-%.

Der Seitenabzug (6) der ersten Kolonne wurde unter Druckerhöhung der zweiten Kolonne (2) zugeführt, die bei 8 bar betrieben wurde. Der Kopfabzug (7) der zweiten Kolonne, der praktisch alles Wasser und und den Großteil der engsiedenden Komponenten enthielt, wurde zur ersten Kolonne (1) zurückgeführt. Ein stark THF angereicherter Strom wird flüssig als Seitenabzug (9) kurz über dem Sumpf entnommen und in Kolonne (3) weitergeleitet.

Der Sumpfabzug von Kolonne (2), der sich als praktisch wasserfrei erwies, wurde über Leitung (8) rückgeführt. Über Kopf (10) der dritten Kolonne (3) wurde Reinst-THF abzogen, während das Sumpfprodukt über eine Pumpe in Kolonne (1) rückgeführt wird.

Die Gehalte an störenden Nebenkomponenten des Rein-THF's sind in Tabelle 1 zusammengefasst.

### Vergleichsbeispiel

Im Gegensatz zu der in DE-A 37 26 805 beschriebenen Fahrweise, erreicht man durch den Seitenabzug (9) an der zweiten Kolonne (2) nach dem vorstehenden erfindungsgemäßen Beispiel 1 eine deutliche Verbesserung in der Abreicherung an Nebenkomponenten im Reinst-THF. Die Trennergebnisse dieser beiden Fahrweisen sind in Tabelle 1 gegenübergestellt.

**Tabelle 1**

| | Butyraldehyd [Gew.-%] | Butylmethylether [Gew.-%] |
|---|---|---|
| wasserhaltiges THF-Gemisch vor Destillation | 0,012 | 0,012 |
| Beispiel 1 | 0,001 | 0,001 |
| Vergleichsbeispiel | 0,005 | 0,003 |

## Patentansprüche

1. Verfahren zur destillativen Reinigung von rohem wasserhaltigen Tetrahydrofuran, bei dem man das rohe Tetrahydrofuran durch drei Destillationskolonnen leitet, Wasser aus dem Sumpf der ersten Kolonne abzieht, wasserhaltiges Tetrahydrofuran vom Kopf der zweiten Kolonne in die erste Kolonne zurückführt, einen Seitenabzug der ersten Kolonne in die zweite Kolonne leitet, das Sumpfprodukt der dritten Kolonne in die erste Kolonne zurückführt, am Kopf der ersten Kolonne ein Destillat entnimmt, das **dadurch gekennzeichnet ist, dass** man einen Seitenabzug der zweiten Kolonne in die dritte Kolonne leitet und das reine Tetrahydrofuran als Kopfprodukt der dritten Kolonne gewinnt.

## Claims

1. A process for distillatively purifying crude water-containing tetrahydrofuran by passing the crude tetrahydrofuran through three distillation columns, withdrawing water from the bottom of the first column, recycling water-containing tetrahydrofuran from the top of the second column into the first column, passing a sidestream of the first column into the second column, recycling the bottom product of the third column into the first column, and withdrawing a distillate at the top of the first column, which comprises passing a sidestream of the second column into the third column and recovering the pure tetrahydrofuran as the top product of the third column.

## Revendications

1. Procédé de purification par distillation de tétrahydrofuranne brut contenant de l'eau, dans lequel on conduit le tétrahydrofuranne brut au travers de trois colonnes de distillation, on soutire l'eau du fond de la première colonne, on recycle du tétrahydrofuranne contenant de l'eau de la tête de la deuxième colonne dans la première colonne, on amène un soutirage latéral de la première colonne dans la deuxième colonne, on recycle le produit du fond de la troisième colonne dans la première colonne, on prélève à la tête de la première colonne un produit de distillation, **caractérisé en qu'**on amène un soutirage latéral de la deuxième colonne dans la troisième colonne et en ce que le tétrahydrofuranne pur est obtenu sous la forme du produit de tête de la troisième colonne.
